# EUROPEAN PATENT APPLICATION

(11) **EP 1 607 103 A1**
(43) Date of publication of application: **21.12.2005**
(21) Application number: 04721714.6
(22) Date of filing: 18.03.2004
(51) Int. Cl.: A61K 45/00, A61P 1/04

(54) **CONCOMITANT DRUG AS THERAPEUTIC AGENT FOR INFLAMMATORY BOWEL DISEASE**

(30) Priority: 20.03.2003 JP 2003077467
(71) Applicant: Eisai Co., Ltd., Tokyo 112-8088 (JP)
(72) Inventor: HORIZOE, Tatsuo, Ushiku-shi, Ibaraki (JP)
(74) Representative: HOFFMANN EITLE
(86) International application number: PCT/JP2004/003662
(87) International publication number: WO 2004/082715

(57) **Abstract**

An object of the present invention is to provide a medicament efficacious for an inflammatory bowel disease such as ulcerative colitis or Crohn's disease. Specifically, it provides a therapeutic agent for inflammatory bowel diseases comprising active ingredient (a) consisting of at least one compound having inflammatory inhibiting activity selected from the group consisting of an aminosalicylic acid derivative, an antiinflammatory glucocorticoid, an immunosuppressive compound, an anti-TNFα antibody, a neurohypophysial hormone and an antiinfective compound, combined with active ingredient (b) consisting of at least one compound having PPARγ agonistic activity, wherein the agent is so configured that the compound (a) and the compound (b) are used simultaneously, separately or every scheduled time.

## Description

### Technical Field

The present invention relates to an agent for treating an inflammatory bowel disease which is useful for treating an inflammatory bowel disease and includes a compound having an agonistic action on PPARγ and another compound having an anti-inflammatory action, such as an aminosalicylic acid derivative, an anti-inflammatory glucocorticoid, a compound having an immunosuppressive action, an anti-TNFα antibody, a pituitary hormone or a compound having an anti-infective action.

### Prior Art

Usefulness of PPARγ agonists for inflammatory bowel diseases such as ulcerative colitis or Crohn's disease has been reported as follows.
(1) US-A 5925657 discloses that a thiazolidine derivative, a PPARγ agonist, inhibits mononuclear leukocytes from producing inflammatory cytokines.
(2) J Exp Med 2001; 193: p. 827-38 and J Clin Invest 1999; 104: p. 383-9 report that single administration of rosiglitazone, a PPARγ agonist, partially inhibits experimental colitis such as murine TNBS-induced colitis or murine DSS-induced colitis.
(3) Other PPARγ agonists are reported to be useful or to be possibly useful for inflammatory bowel diseases such as ulcerative colitis or Crohn's disease (WO 02/100812 and WO 02/080899).

An inflammatory bowel disease such as ulcerative colitis or Crohn's disease is fundamentally treated by a symptomatic treatment with an aminosalicylic acid derivative or an anti-inflammatory glucocorticoid. Novel treatments using an immunosuppressive drug or a preparation containing an anti-TNF-α antibody have received attention in recent years (Nippon Rinsho (in Japanese; Japanese Journal of Clinical Medicine), 2002; 60(3): 480-486).

However, no medicament for treating inflammatory bowel diseases which is satisfactory from the viewpoints of treatment efficacy and adverse drug actions has been found (Nippon Rinsho (in Japanese; Japanese Journal of Clinical Medicine), 2002; 60(3): 531-538).

A combination of Rosiglitazone, a PPARγ agonist and an aminosalicylic acid derivative, an anti-inflammatory glucocorticoid and/or an immunosuppressive drug has been reported. This report, however, fails to disclose comparison between the combination treatment and the single administration of the PPARγ agonist and fails to describe effects of the combination treatment (Am J Gastroenterol 2001; 96: 3323-8).

Accordingly, an object of the present invention is to provide a medicament that is more efficacious on an inflammatory bowel disease such as ulcerative colitis or Crohn's disease.

### Disclosure of Invention

Under these circumstances, the present inventors have found a medicament having satisfactory effects on an inflammatory bowel disease such as ulcerative colitis or Crohn's disease by using a combination of a compound having an agonistic action on PPARγ and another compound having an anti-inflammatory action such as an aminosalicylic acid derivative, an anti-inflammatory glucocorticoid, a compound having an immunosuppressive action, an anti-TNFα antibody, a pituitary hormone and/or a compound having an anti-infective action. The present invention has been achieved based on these findings.

Specifically, the present invention relates to:
(1) an agent for treating an inflammatory bowel disease comprising a combination of (a) at least one compound having an anti-inflammatory action and being selected from the group consisting of an aminosalicylic acid derivative, an anti-inflammatory glucocorticoid, a compound having an immunosuppressive action, an anti-TNFα antibody, a pituitary hormone and a compound having an anti-infective action as an active ingredient and (b) at least one compound having a PPARγ agonistic action as an active ingredient, wherein the agent is so configured that the compound (a) and the compound (b) are used simultaneously, separately or every scheduled time;
(2) an agent for treating an inflammatory bowel disease comprising a combination of (a) at least one compound having an anti-inflammatory action and being selected from the group consisting of an aminosalicylic acid derivative, an anti-inflammatory glucocorticoid, a compound having an immunosuppressive action and an anti-TNFα antibody as an active ingredient and (b) at least one compound having a PPARγ agonistic action as an active ingredient, wherein the agent is so configured that the compound (a) and the compound (b) are used simultaneously, separately or every scheduled time;
(3) an agent for treating an inflammatory bowel disease comprising a combination of (a) at least one compound having an anti-inflammatory action and being selected from the group consisting of an aminosalicylic acid derivative and a compound having an immunosuppressive action as an active ingredient and (b) at least one compound having a PPARγ agonistic action as an active ingredient, wherein the agent is so configured that the compound (a) and the compound (b) are used simultaneously, separately or every scheduled time;
(4) the agent for treating an inflammatory bowel disease described in the above (1), wherein the compound having a PPARγ agonistic action is a compound having a PPARγ agonistic action for use in leukocytapheresis treatment or granulocytapheresis treatment;
(4-2) An agent for treating an inflammatory bowel disease, which comprises a compound having a PPARγ agonistic action and being used in leukocytapheresis treatment or granulocytapheresis treatment;
(5) the agent for treating an inflammatory bowel disease described in any one of the above (1) to (4), wherein the compound having a PPARγ agonistic action is a compound selected from the group consisting of:
   (1) 2-isopropoxy-3-[3-([4-(trifluoromethyl)benzyl]-oxyethanimidoyl)phenyl]propanoic acid,
   (2) 3-{3-[3-(2,4-dichlorophenoxy)-2(S)-hydroxypropoxy]phenyl}-2(S)-isopropoxypropanoic acid,
   (3) 3-(3-{2(R)-hydroxy-3-[4-chlorophenoxy]propoxy}phenyl)-2(S)-isopropoxypropanoic acid,
   (4) 3-{3-[3-(2,4-dichlorophenoxy)-2(R)-hydroxypropoxy]phenyl}-2(S)-isopropoxypropanoic acid,
   (5) 3-{3-[3-(2,4-dichlorophenoxy)-2(R)-fluoropropoxy]phenyl}-2(S)-isopropoxypropanoic acid,
   (6) 3-{3-[3-(4-chlorophenoxy)-2(S)-fluoropropoxy]phenyl}-2(S)-isopropoxypropanoic acid,
   (7) 3-{3-[3-(4-chloro-2-cyanophenoxy)-2(R)-fluoropropoxy]phenyl}-2(S)-isopropoxypropanoic acid,
   (8) 3-{3-[3-(2,4-dichlorophenoxy)-2(S)-fluoropropoxy]phenyl}-2(S)-isopropoxypropanoic acid,
   (9) 3-{3-[3-(4-chlorophenoxy)-2(R)-fluoropropoxy]phenyl}-2(S)-isopropoxypropanoic acid,
   (10) 3-{3-[3-(4-chloro-2-cyanophenoxy)-2(S)-fluoropropoxy]phenyl}-2(S)-isopropoxypropanoic acid,
   (11) 2(S)-isopropoxy-3-{3-[(4-trifluoromethylbenzyloxycarbonylamino)methyl]phenyl}-propanoic acid,
   (12) 2(S)-isopropoxy-3-{3-[(3-trifluoromethylbenzyloxycarbonylamino)methyl]phenyl}-propanoic acid,
   (13) 2(S)-isopropoxy-3-{3-[(4-trifluoromethoxybenzyloxycarbonylamino)methyl]phenyl}-propanoic acid,
   (14) 3-(3-{[3-trifluoromethoxybenzyloxycarbonylamino]-methyl}phenyl)-2(S)-isopropoxypropanoic acid,
   (15) 3-{[3-(2,4-dichlorophenyl)carbamoyloxymethyl-4-ethoxy]phenyl}-2-isopropoxypropanoic acid,
   (16) 3-({4-[5-(benzo[1,3]dioxolyl)]carbamoyloxymethyl}-phenyl)-2-isopropoxypropanoic acid,
   (17) 3-{3-[3-(4-chlorophenyl)-2-propynyloxy]phenyl}-2(S)-isopropoxypropanoic acid,
   (18) 3-{[3-(2,4-dichlorophenyl)carbamoyloxymethyl-4-ethoxy]phenyl}-2(S)-isopropoxypropanoic acid,
   (19) 3-(3-{2(R)-hydroxy-3-[2-bromo-4-methylphenoxy]-propoxy}phenyl)-2(S)-isopropoxypropanoic acid,
   (20) 3-{[4-(4-ethoxyphenyl)carbamoyloxymethyl]phenyl}-2-isopropoxypropanoic acid,
   (21) 2-isopropoxy-3-[4-(2-{[4-(trifluoromethyl)phenyl]-carbamoyloxy}ethyl)phenyl]propanoic acid,
   (22) 3-[3-([2,4-dichlorobenzoyl]aminomethyl)-4-methoxyphenyl]-2(S)-isopropoxypropanoic acid,
   (23) 3-[3-([2-fluoro-4-(trifluoromethyl)benzoyl]-aminomethyl)-4-methoxyphenyl]-2(S)-isopropoxypropanoic acid,
   (24) 2-ethoxy-3-(2-2-[4-(trifluoromethyl)phenoxy]ethoxy}-4-pyridyl)propanoic acid,
   (25) 3-(2-{2-[4-(tert-butyl)phenoxy]ethoxy}-4-pyridyl)-2-ethoxypropanoic acid,
   (26) 3-(3-[(2-chloro-4-propoxybenzoyl)amino]methyl-2,4-dimethoxyphenyl)-2-isopropoxypropanoic acid,
   (27) 3-(7-[(2,4-dichlorobenzoyl)amino]methylbenzo[b]furan-5-yl)-2-isopropoxypropanoic acid,
   (28) 3-(7-[(2-chloro-4-propoxybenzoyl)amino]methyl-2,3-dihydrobenzo[b]furan-5-yl)-2-isopropoxypropanoic acid,
   (29) 2-(3-{[(2-chloro-4-propoxybenzoyl)amino]methyl}-4-ethoxybenzyl)tetrahydro-2-furancarboxylic acid,
   (30) 2-(3-{[(2-chloro-4-propoxybenzoyl)amino]methyl}-4-ethoxybenzyl)tetrahydro-2-furancarboxylic acid,
   (31) pioglitazone,
   (32) netoglitazone,
   (33) tesaglitazar,
   (34) 5-(2,4-dioxothiazolidin-5-ylmethyl)-2-methoxy-N-(4-trifluoromethylbenzyl)benzamide,
   (35) 5-[4-(6-methoxy-1H-benzimidazol-2-ylmethoxy)benzyl]-thiazolidine-2,4-dione,
   (36) 4-[4-(5-methyl-2-phenyloxazol-4-ylmethoxy)-benzyloxyimino]-4-phenylbutyric acid and
   (37) (4-methoxyphenoxycarbonyl-[4-[2-(5-methyl-2-phenyloxazo-4-yl)ethoxy]benzyl]amino)acetic acid,
   a salt thereof or a hydrate of them;
(6) the agent for treating an inflammatory bowel disease described in any one of the above (1) to (4), wherein the compound having a PPARγ agonistic action is a compound selected from the group consisting of:
   (1) 2-isopropoxy-3-[3-([4-(trifluoromethyl)benzyl]-oxyethanimidoyl)phenyl]propanoic acid,
   (2) 3-{3-[3-(2,4-dichlorophenoxy)-2(R)-fluoropropoxy]phenyl}-2(S)-isopropoxypropanoic acid,
   (3) 3-{3-[3-(4-chlorophenoxy)-2(R)-fluoropropoxy]phenyl}-2(S)-isopropoxypropanoic acid,
   (4) 3-{3-[3-(4-chloro-2-cyanophenoxy)-2(S)-fluoropropoxy]phenyl}-2(S)-isopropoxypropanoic acid,
   (5) 2(S)-isopropoxy-3-{3-[(4-trifluoromethoxybenzyloxycarbonylamino)methyl]phenyl}-propanoic acid,
   (6) 3-(3-{[3-trifluoromethoxybenzyloxycarbonylamino]-methyl}phenyl)-2(S)-isopropoxypropanoic acid,
   (7) pioglitazone,
   (8) netoglitazone,
   (9) tesaglitazar,
   (10) 5-(2,4-dioxothiazolidin-5-ylmethyl)-2-methoxy-N-(4-trifluoromethylbenzyl)benzamide,
   (11) 5-[4-(6-methoxy-1H-benzimidazol-2-ylmethoxy)benzyl]-thiazolidine-2,4-dione,
   (12) 4-[4-(5-methyl-2-phenyloxazol-4-ylmethoxy)-benzyloxyimino]-4-phenylbutyric acid and
   (13) (4-methoxyphenoxycarbonyl-[4-[2-(5-methyl-2-phenyloxazo-4-yl)ethoxy]benzyl]amino)acetic acid,
   a salt thereof or a hydrate of them;
(7) the agent for treating an inflammatory bowel disease described in any one of the above (1) to (4), wherein the compound having a PPARγ agonistic action is a compound selected from the group consisting of:
   (1) 2-isopropoxy-3-[3-([4-(trifluoromethyl)benzyl]-oxyethanimidoyl)phenyl]propanoic acid,
   (2) 3-{3-[3-(2,4-dichlorophenoxy)-2(S)-hydroxypropoxy]phenyl}-2(S)-isopropoxypropanoic acid,
   (3) 3-(3-{2(R)-hydroxy-3-[4-chlorophenoxy]propoxy}phenyl)-2(S)-isopropoxypropanoic acid,
   (4) 3-{3-[3-(2,4-dichlorophenoxy)-2(R)-hydroxypropoxy]phenyl}-2(S)-isopropoxypropanoic acid,
   (5) 3-{3-[3-(2,4-dichlorophenoxy)-2(R)-fluoropropoxy]phenyl}-2(S)-isopropoxypropanoic acid,
   (6) 3-{3-[3-(9-chlorophenoxy)-2(S)-fluoropropoxy]phenyl}-2(S)-isopropoxypropanoic acid,
   (7) 3-{3-[3-(4-chloro-2-cyanophenoxy)-2(R)-fluoropropoxy]phenyl}-2(S)-isopropoxypropanoic acid,
   (8) 3-{3-[3-(2,4-dichlorophenoxy)-2(S)-fluoropropoxy]phenyl}-2(S)-isopropoxypropanoic acid,
   (9) 3-{3-[3-(4-chlorophenoxy)-2(R)-fluoropropoxy]phenyl}-2(S)-isopropoxypropanoic acid,
   (10) 3-{3-[3-(4-chloro-2-cyanophenoxy)-2(S)-fluoropropoxy]-phenyl}-2(S)-isopropoxypropanoic acid,
   (11) 2(S)-isopropoxy-3-{3-[(4-trifluoromethylbenzyloxycarbonylamino)methyl]phenyl}-propanoic acid,
   (12) 2(S)-isopropoxy-3-{3-[(3-trifluoromethylbenzyloxycarbonylamino)methyl]phenyl}-propanoic acid,
   (13) 2(S)-isopropoxy-3-{3-[(4-trifluoromethoxybenzyloxycarbonylamino)methyl]phenyl}-propanoic acid,
   (14) 3-(3-{[3-trifluoromethoxybenzyloxycarbonylamino]-methyl} phenyl)-2(S)-isopropoxypropanoic acid,
   (15) 3-{[3-(2,4-dichlorophenyl)carbamoyloxymethyl-4-ethoxy]phenyl}-2-isopropoxypropanoic acid,
   (16) 3-({4-[5-(benzo[1,3]dioxolyl)]carbamoyloxymethyl}-phenyl)-2-isopropoxypropanoic acid,
   (17) 3-{3-[3-(4-chlorophenyl)-2-propynyloxy]phenyl}-2(S)-isopropoxypropanoic acid,
   (18) 3-{[3-(2,4-dichlorophenyl)carbamoyloxymethyl-4-ethoxy]phenyl}-2(S)-isopropoxypropanoic acid,
   (19) 3-(3-{2(R)-hydroxy-3-[2-bromo-4-methylphenoxy]propoxy}phenyl)-2(S)-isopropoxypropanoic acid,
   (20) 3-{[4-(4-ethoxyphenyl)carbamoyloxymethyl]phenyl}-2-isopropoxypropanoic acid,
   (21) 2-isopropoxy-3-[4-(2-{[4-(trifluoromethyl)phenyl]-carbamoyloxy}ethyl)phenyl]propanoic acid,
   (22) 3-[3-([2,4-dichlorobenzoyl]aminomethyl)-4-methoxyphenyl]-2(S)-isopropoxypropanoic acid,
   (23) 3-[3-([2-fluoro-4-(trifluoromethyl)benzoyl]-aminomethyl)-4-methoxyphenyl]-2(S)-isopropoxypropanoic acid,
   (24) 2-ethoxy-3-(2-{2-[4-(trifluoromethyl)phenoxy]ethoxy}-4-pyridyl)propanoic acid,
   (25) 3-(2-{2-[4-(tert-butyl)phenoxy]ethoxy}-4-pyridyl)-2-ethoxypropanoic acid,
   (26) 3-(3-[(2-chloro-4-propoxybenzoyl)amino]methyl-2,4-dimethoxyphenyl)-2-isopropoxypropanoic acid,
   (27) 3-(7-[(2,4-dichlorobenzoyl)amino]methylbenzo[b]furan-5-yl)-2-isopropoxypropanoic acid,
   (28) 3-(7-[(2-chloro-4-propoxybenzoyl)amino]methyl-2,3-dihydrobenzo[b]furan-5-yl)-2-isopropoxypropanoic acid,
   (29) 2-(3-{[(2-chloro-4-propoxybenzoyl)amino]methyl}-4-ethoxybenzyl)tetrahydro-2-furancarboxylic acid and
   (30) 2-(3-{[(2-chloro-4-propoxybenzoyl)amino]methyl}-4-ethoxybenzyl)tetrahydro-2-furancarboxylic acid,
   a salt thereof or a hydrate of them;
(8) the agent for treating an inflammatory bowel disease described in any one of the above (1) to (4), wherein the compound having a PPARγ gonistic action is a compound selected from the group consisting of:
   (1) 2-isopropoxy-3-[3-([4-(trifluoromethyl)benzyl]-oxyethanimidoyl)phenyl]propanoic acid,
   (2) 3-{3-[3-(2,4-dichlorophenoxy)-2(R)-fluoropropoxy]phenyl}-2(S)-isopropoxypropanoic acid,
   (3) 3-{3-[3-(4-chlorophenoxy)-2(R)-fluoropropoxy]phenyl}-2(S)-isopropoxypropanoic acid,
   (4) 3-{3-[3-(4-chloro-2-cyanophenoxy)-2(S)-fluoropropoxy]phenyl}-2(S)-isopropoxypropanoic acid,
   (5) 2(S)-isopropoxy-3-{3-[(4-trifluoromethoxybenzyloxycarbonylamino)methyl]phenyl}-propanoic acid and
   (6) 3-(3-{[3-trifluoromethoxybenzyloxycarbonylamino]-methyl}phenyl)-2(S)-isopropoxypropanoic acid, a salt thereof or a hydrate of them;
(8-2) the agent for treating an inflammatory bowel disease described in any one of the above (1) to (4), wherein the compound having a PPARγ gonistic action is a compound selected from the group consisting of:
   3-{3-[3-(4-chloro-2-cyanophenoxy)-2(S)-fluoropropoxy]phenyl}-2(S)-isopropoxypropanoic acid and
   3-(3-{[3-trifluoromethoxybenzyloxycarbonylamino]methyl}-phenyl)-2(S)-isopropoxypropanoic acid, a salt thereof or a hydrate of them;
(9) the agent for treating an inflammatory bowel disease described in any one of the above (1) to (4), wherein the compound having a PPARγ agonistic action is 3-{3-[3-(4-chloro-2-cyanophenoxy)-2(S)-fluoropropoxy]phenyl}-2(S)-isopropoxypropanoic acid, a salt thereof or a hydrate of them.
(10) the agent for treating an inflammatory bowel disease described in any one of the above (1) to (3), wherein the aminosalicylic acid derivative is:
   (1) sulfasalazine,
   (2) mesalazine,
   (3) olsalazine or
   (4) balsalazide;
(11) the agent for treating an inflammatory bowel disease described in the above (1) or (2), wherein the anti-inflammatory glucocorticoid is:
   (1) prednisolone,
   (2) betamethasone,
   (3) hydrocortisone,
   (4) cortisone acetate,
   (5) methylprednisolone,
   (6) prednisone or
   (7) budesonide;
(12) the agent for treating an inflammatory bowel disease described in any one of the above (1) to (3), wherein the compound having an immunosuppressive action is:
   (1) cyclosporin,
   (2) azathioprine,
   (3) 6-mercaptopurine,
   (4) tacrolimus or
   (5) methotrexate;
(13) the agent for treating an inflammatory bowel disease described in the above (1) or (2), wherein the anti-TNFα antibody is an antibody contained in:
   (1) infliximab,
   (2) etanercept,
   (3) CDP-571,
   (4) adalimumab or
   (5) CDP-870;
(14) the agent for treating an inflammatory bowel disease described in the above (1), wherein the compound having an anti-infective action is:
   (1) metronidazole,
   (2) clarithromycin,
   (3) tobramycin,
   (4) ciprofloxacin hydrochloride,
   (5) ampicillin,
   (6) cefazolin,
   (7) ofloxacine or
   (8) levofloxacin;
(15) the agent for treating an inflammatory bowel disease described in the above (1), wherein the pituitary hormone is tetracosactide acetate;
(16) the agent for treating an inflammatory bowel disease described in the above (2), wherein the group consisting of an aminosalicylic acid derivative, an anti-inflammatory glucocorticoid, a compound having an immunosuppressive action and an anti-TNFα antibody is the group consisting of sulfasalazine, mesalazine, prednisolone, betamethasone, hydrocortisone, cortisone acetate, methylprednisolone, prednisone, cyclosporin and tacrolimus;
(17) the agent for treating an inflammatory bowel disease described in the above (3), wherein one compound having an anti-inflammatory action and being selected from the group consisting of an aminosalicylic acid derivative and a compound having an immunosuppressive action is sulfasalazine or cyclosporine;
(18) an agent for treating an inflammatory bowel disease, which comprises a combination of (a) a compound having an anti-inflammatory action and being selected from the group consisting of sulfasalazine and cyclosporin as an active ingredient and (b) a compound having a PPARγ agonistic action and being 3-{3-[3-(4-chloro-2-cyanophenoxy)-2(S)-fluoropropoxy]phenyl}-2(S)-isopropoxypropanoic acid, a salt thereof or a hydrate of them as an active ingredient, wherein the agent is so configured that the compound (a) and the compound (b) are used simultaneously, separaely or every scheduled time;
(19) the agent described in any one of the above (1) to (17), wherein the inflammatory bowel disease is ulcerative colitis;
(20) the agent described in any one of the above (1) to (17), wherein the inflammatory bowel disease is Crohn's disease;
(21) a kit for treating an inflammatory bowel disease, which comprises an agent for treating an inflammatory bowel disease comprising a combination of (a) at least one compound having an anti-inflammatory action and being selected from the group consisting of an aminosalicylic acid derivative, an anti-inflammatory glucocorticoid, a compound having an immunosuppressive action, an anti-TNFα antibody, a pituitary hormone and a compound having an anti-infective action as an active ingredient and (b) at least one compound having a PPARγ agonistic action as an active ingredient, wherein the agent is so configured that the compound (a) and the compound (b) are used simultaneously, separately or every scheduled time.
(22) a method for treating an inflammatory bowel disease, which comprises administering an effective amount of the agent described in any one of the above (1) to (18) to a patient; or
(23) use of the agent described in any one of the above (1) to (18), for producing an agent for treating an inflammatory bowel disease.

The present invention provides an agent for treating an inflammatory bowel disease comprising the combination of the compound (a) and the compound (b) described in the above-mentioned (1).

The present invention also provides use of the combination of the compound (a) and the compound (b) described in the above-mentioned (1), for producing an agent for treating an inflammatory bowel disease.

The present invention also provides a method for treating an inflammatory bowel disease, which comprises administering a pharmacologically effective amount of the combination of the compound (a) and the compound (b) described in the above-mentioned (1) to a patient.

As the "combination of the compound (a) and the compound (b)", administering the compound (a) and the compound (b) simultaneously, separately or every scheduled time is preferred.

In the present description, the "agent for treating an inflammatory bowel disease comprising a combination of (a) at least one compound having an anti-inflammatory action and being selected from the group consisting of an aminosalicylic acid derivative, an anti-inflammatory glucocorticoid, a compound having an immunosuppressive action, an anti-TNFα antibody, a pituitary hormone and a compound having an anti-infective action as an active ingredient and (b) at least one compound having a PPARγ agonistic action as an active ingredient, wherein the agent is so configured that the compound (a) and the compound (b) are used simultaneously, separately or every scheduled time" means an agent for treating an inflammatory bowel disease, which comprises a combination of one or more of the compounds (a) and one or more of the compounds (b).
The dose, frequency of administration, administration rout, dosage form and administration timing of each compound comprised in the agent for treating an inflammatory bowel disease can be independently and appropriately selected and employed depending typically on the age, body weight, sex, degree of symptom of the patient, and the type and dose of the other agent used.

In the present description, the "agent for treating an inflammatory bowel disease comprising a combination of (a) at least one compound having an anti-inflammatory action and being selected from the group consisting of an aminosalicylic acid derivative, an anti-inflammatory glucocorticoid, a compound having an immunosuppressive action, an anti-TNFα antibody, a pituitary hormone and a compound having an anti-infective action as an active ingredient and (b) at least one compound having a PPARγ agonistic action as an active ingredient, wherein the agent is so configured that the compound (a) and the compound (b) are used simultaneously, separately or every scheduled time" is preferably an "agent for treating an inflammatory bowel disease comprising a combination of (a) at least one compound having an anti-inflammatory action and being selected from the group consisting of an aminosalicylic acid derivative, an anti-inflammatory glucocorticoid, a compound having an immunosuppressive action, an anti-TNFα antibody, a pituitary hormone and a compound having an anti-infective action as an active ingredient and (b) a compound having a PPARγ agonistic action as an active ingredient, wherein the agent is so configured that the compound (a) and the compound (b) are used simultaneously, separately or every scheduled time".

The "agent for treating an inflammatory bowel disease comprising a combination of (a) at least one compound having an anti-inflammatory action and being selected from the group consisting of an aminosalicylic acid derivative, an anti-inflammatory glucocorticoid, a compound having an immunosuppressive action and an anti-TNFα antibody as an active ingredient and (b) at least one compound having a PPARγ agonistic action as an active ingredient, wherein the agent is so configured that the compound (a) and the compound (b) are used simultaneously, separately or every scheduled time" in the present description is preferably an "agent for treating an inflammatory bowel disease comprising a combination of (a) a compound having an anti-inflammatory action and being selected from the group consisting of an aminosalicylic acid derivative, an anti-inflammatory glucocorticoid, a compound having an immunosuppressive action and an anti-TNFα antibody as an active ingredient and (b) a compound having a PPARγ agonistic action as an active ingredient, wherein the agent is so configured that the compound (a) and the compound (b) are used simultaneously, separately or every scheduled time".

The "agent for treating an inflammatory bowel disease comprising a combination of (a) at least one compound having an anti-inflammatory action and being selected from the group consisting of an aminosalicylic acid derivative and a compound having an immunosuppressive action as an active ingredient and (b) at least one compound having a PPARγ agonistic action as an active ingredient, wherein the agent is so configured that the compound (a) and the compound (b) are used simultaneously, separately or every scheduled time" in the present description is preferably an "agent for treating an inflammatory bowel disease comprising a combination of (a) a compound having an anti-inflammatory action and being selected from the group consisting of an aminosalicylic acid derivative and a compound having an immunosuppressive action as an active ingredient and (b) a compound having a PPARγ agonistic action as an active ingredient, wherein the agent is so configured that the compound (a) and the compound (b) are used simultaneously, separately or every scheduled time".

Optimum dosage forms of the respective compounds in the compounds (a) and (b), can be independently and appropriately selected from, for example, tablets; powders; granules; capsules; syrups; troches; inhalants; suppositories; clysters; injections such as intravascular injections, subcutaneous injections, intramuscular injections and drip infusions; ointments; ophthalmic ointments; eye drops; nasal drops; ear drops; cataplasms; and lotions.

Optimum administration modes of the respective compounds in the compounds (a) and (b) can be independently and appropriately selected from dosage forms such as oral administration; injections such as intravascular injections, subcutaneous injections, intramuscular injections and drip infusions; integumentary administration; local administration using, for example, suppositories, enema solutions or enema agents; and inhalation administration.

The respective compounds in the compounds (a) and (b) can be administered (used) at any timings. For example, they can be administered (used) simultaneously, separately (at some time interval) or every scheduled time. The optimum administration timings (e.g., daily dosage frequency and dose) of the respective compounds can be independently and appropriately set.

The doses of the respective compounds in the compounds (a) and (b) significantly vary depending typically on the type of target disease, degree of symptom, age, sex and drug sensibility of the patient. Generally, each compound may be administered to an adult in one to several divided doses at a daily dose of about 0.03 to 6000 mg, preferably 0.1 to 500 mg and more preferably 0.1 to 100 mg. For injection administration, the dose is generally preferably about 1 µg/kg to 3000 µg/kg, and more preferably about 3 µg/kg to 1000 µg/kg.

The administration route of the agent for treating an inflammatory bowel disease of the present invention is not specifically limited, as long as the compound (a) and the compound (b) are in combination in administration. Examples of such administration modes are:
(1) an agent for treating an inflammatory bowel disease prepared by formulating the respective compounds into a single active ingredient preparation, or
(2) a combination of medicaments prepared by formulating the respective compounds independently.

In the formulation, generally used fillers, binders, disintegrators, lubricants, coloring agents and flavoring agents, as well as stabilizers, emulsifiers, absorbefacients, surfactants, pH adjusting agents, antiseptics and antioxidants according to necessity can be used. They can be formulated according to a conventional procedure using components generally used as raw materials for pharmaceutical preparations. Examples of such components include (1) animal or vegetable oils such as soybean oil, beef tallow or synthetic glycerides; (2) hydrocarbons such as liquid paraffins, squalane or solid paraffins; (3) ester oils such as octyldodecyl myristate or isopropyl myristate; (4) higher alcohols such as cetostearyl alcohol or behenyl alcohol; (5) silicone resins; (6) silicone oils; (7) surfactants such as polyoxyethylene fatty acid esters, sorbitan fatty acid esters, glycerin fatty acid esters, polyoxyethylene sorbitan fatty acid esters, polyoxyethylene hydrogenated castor oils or polyoxyethylene-polyoxypropylene block copolymers; (8) water-soluble polymers such as hydroxyethyl cellulose, polyacrylic acid, carboxyvinyl polymers, polyethylene glycol, polyvinylpyrrolidone or methylcellulose; (9) lower alcohols such as ethanol or isopropanol; (10) polyhydric alcohols such as glycerol, propylene glycol, dipropylene glycol or sorbitol; (11) sugars such as glucose or sucrose; (12) inorganic powders such as silicic anhydride, magnesium aluminium silicate or aluminium silicate; and (13) purified water.

Examples of the fillers are lactose, corn starch, sucrose, glucose, mannitol, sorbitol, crystalline cellulose and silicon dioxide; examples of the binders are polyvinyl alcohol, polyvinyl ether, methylcellulose, ethylcellulose, gum arabic, gum tragacanth, gelatin, shellac, hydroxypropyl cellulose, hydroxypropylmethyl cellulose, polyvinylpyrrolidone, polypropylene glycol-polyoxyethylene block polymers, meglumine, calcium citrate, dextrin and pectin; examples of the disintegrators are starch, agar, gelatin powder, crystalline cellulose, calcium carbonate, sodium hydrogencarbonate, calcium citrate, dextrin, pectin and carboxymethylcellulose calcium; examples of the lubricants are magnesium stearate, talc, polyethylene glycol, silica and hydrogenated vegetable oils; the coloring agents can be any coloring agents which are approved to add to pharmaceutical preparations; examples of the flavoring agents are cocoa powder, menthol, aromatic powder, peppermint oil, borneol and cinnamon powder; the antioxidants can be any antioxidants which are approved to add to pharmaceutical preparations such as ascorbic acid or α-tocopherol.

As oral preparations, the active ingredients are compounded with a filler, and if necessary, a binder, disintegrator, lubricant, coloring agent, flavoring agent and other components, and the resulting mixture is formulated according to a conventional procedure into, for example, a powder, fine granules, granules, tablets, coated tablets or capsules. The tablets and granules can be appropriately coated with, for example, sugar or gelatin, or other according to necessity. Liquid formulations such as syrups or injection preparations can be prepared according to a conventional procedure, by adding a pH adjusting agent, solubilizer and isotonizing agent, and if necessary, a solubilizing agent, stabilizer, buffer, suspending agent, antioxidant and other components. The liquid formulations can also be formed into freeze-dried products. Preferred examples of the suspending agents are methylcellulose, polysorbate 80, hydroxyethyl cellulose, gum arabic, powdered tragacanth, carboxymethylcellulose sodium and polyoxyethylene sorbitan monolaurate; preferred examples of the solubilizers are polyoxyethylene hydrogenated caster oil, polysorbate 80, nicotinamide and polyoxyethylene sorbitan monolaurate; preferred examples of the stabilizers are sodium sulfite, sodium metasulfite and ether; preferred examples of the preservatives are methyl p-hydroxybenzoate, ethyl p-hydroxybenzoate, sorbic acid, phenol, cresol and chlorocresol. External preparations can be produced according to a conventional procedure not specifically limited. Base materials for use herein can be any raw materials generally used in, for example, pharmaceutical preparations, quasi drugs and cosmetics. Such raw materials include, for example, animal or vegetable oils, mineral oils, ester oils, waxes, higher alcohols, fatty acids, silicone oils, surfactants, phospholipids, alcohols, polyhydric alcohols, water-soluble polymers, clay minerals and purified water. Where necessary, any of pH adjusting agents, antioxidants, chelating agents, antiseptics and antimolds, coloring agents and flavors can be added. In addition, components having a differentiation-inducing action, blood-flow accelerators, bactericides, anti-inflammatory agents, cell activators, vitamins, amino acids, humectants, keratolytic agents and other components can be added according to necessity.

The term "inflammatory bowel disease" as used in the present description means an inflammatory bowel disease such as ulcerative colitis, Crohn's disease, infective colitis, drug-induced colitis, ischaemic colitis, radiation colitis, intestinal tuberculosis or enteric syphilis.

The "aminosalicylic acid derivative" as used in the present description means, for example:
(1) Sulfasalazine (The Japanese Pharmacopoeia Fourteenth Edition, C-1374) (The Merck Index, 13th Edition, No. 9028),
(2) Mesalazine (The Merck Index, 13th Edition, No. 5931),
(3) Olsalazine (The Merck Index, 13th Edition, No. 6911) and/or
(4) Balsalazide (The Merck Index, 13th Edition, No. 947).

The "anti-inflammatory glucocorticoid" as used in the present description means a hormone relating to carbohydrate metabolism among steroid hormones secreted from the adrenal cortex, as well as synthetic substances having a similar action, such as an adrenal corticosteroid or a synthetic adrenal corticosteroid.

Specifically, the anti-inflammatory glucocorticoid means, for example:
(1) prednisolone (The Japanese Pharmacopoeia Fourteenth Edition, C-2537)
(2) betamethasone (The Japanese Pharmacopoeia Fourteenth Edition, C-2620, C-1143 or C-1527)
(3) hydrocortisone (The Japanese Pharmacopoeia Fourteenth Edition, C-2319, C-1301, C-1354 or C-2988)
(4) cortisone acetate (The Japanese Pharmacopoeia Fourteenth Edition, C-1339)
(5) methylprednisolone (The Japanese Pharmacopoeia Fourteenth Edition, C-2871)
(6) prednisone (The Japanese Pharmacopoeia Fourteenth Edition, B-905) and
(7) budesonide (The Merck Index, 13th Edition, No. 1454).

The "compound having an immunosuppressive action" as used in the present description means, for example:
(1) cyclosporin (The Japanese Pharmacopoeia Fourteenth Edition, C-1445)
(2) azathioprine (The Japanese Pharmacopoeia Fourteenth Edition, C-23 or 28)
(3) 6-mercaptopurine (The Japanese Pharmacopoeia Fourteenth Edition, C-2920)
(4) tacrolimus (The Merck Index, 13th Edition, No. 9117) and
(5) methotrexate (The Japanese Pharmacopoeia Fourteenth Edition, C-2890).

The "anti-TNF-α antibody" as used in the present description means an antibody contained in:
(1) infliximab (The Merck Index, 13th Edition, No. 4995) (CAS No. 170277-31-3),
(2) etanercept (The Merck Index, 13th Edition, No. 3747),
(3) CDP-571 (Gastroenterology, 2001; vol. 120, p. 1330-1338),
(4) adalimumab (CAS No. 331731-18-1),
(5) CDP-870 (Rheumatology, 2002; vol. 41, p. 1133-1137) (Nippon Rinsho (in Japanese; Japanese Journal of Clinical Medicine), 2002; 60(3): 480-486) etc.

The "pituitary hormone preparation" as used in the present description means a therapeutic agent comprising a pituitary hormone such as Tetracosactide acetate.

The "compound having an anti-infective action" as used in the present description means, for example:
(1) metronidazole (The Japanese Pharmacopoeia Fourteenth Edition, C-2896),
(2) clarithromycin (The Japanese Pharmacopoeia Fourteenth Edition, C-1195),
(3) tobramycin (The The Japanese Pharmacopoeia Fourteenth Edition, C-2033),
(4) ciprofloxacin hydrochloride (The Merck Index, 13th Edition, No. 2337),
(5) ampicillin (The Japanese Pharmacopoeia Fourteenth Edition, C-190, C-192 or 193),
(6) cefazolin (The Japanese Pharmacopoeia Fourteenth Edition, C-1756 or 1762),
(7) ofloxacin (CAS. No. 82419-36-1) and
(8) levofloxacin (CAS. No. 100986-85-4).

The "leukocytapheresis treatment" as used in the present description means a membranous leukocytapheresis treatment, in which leukocytes in the blood exteriorized from a patient are brought into contact with a leukopheretic device filled with ultrafine polyester fibers (Cellsorba, Asahi Medical Co., Ltd.) to thereby remove leukocytes in a manner nonspecific to fractions (Nippon Rinsho (in Japanese; Japanese Journal of Clinical Medicine), 1999; 57(11): 2496-2502).

The "granulocytapheresis treatment" as used in the present description means a method for selectively adsorbing and removing granulocytes by allowing the blood exteriorized from a patient to pass through a column (G-1 Column) filled with cellulose acetate beads (Nippon Rinsho (in Japanese; Japanese Journal of Clinical Medicine), 1999; 57(11): 2496-2502).

Sulfasalazine as used in the present description means 2-hydroxy-5-[[4-[(2-pyridinylamino)sulfonyl]phenyl]-azo]benzoic acid, i.e., the compound represented by: 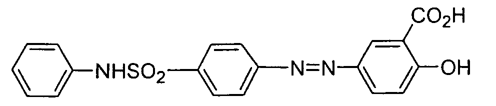 (The Japanese Pharmacopoeia Fourteenth Edition, C-1374) (The Merck Index, 13th Edition, No. 9028), a salt thereof or a hydrate of them.

When sulfasalazine is used in "the agent for treating an inflammatory bowel disease for concomitant use" in the present description, the dose, administration frequency, administration route, dosage form and administration timing of sulfasalazine and other parameters can be selected according typically to the age, body weight, sex and degree of symptom of the patient, as well as the type and dose of another therapeutic agent used. Specifically, it can be administered, for example, by:
(1) oral administration: 1000 to 6000 (mg/day)
(2) local administration (suppository): 1000 to 2000 (mg/day)

Cyclosporine as used in the present description means cyclosporin A described in The Japanese Pharmacopoeia Fourteenth Edition, C-1445, a salt thereof or a hydrate of them (The Merck Index 13th Edition, No. 2781).

When cyclosporin is used in "the agent for treating an inflammatory bowel disease for concomitant use" in the present description, the dose, administration frequency, administration rout, dosage form and administration timing of cyclosporin can be appropriately selected according typically to the age, body weight, sex and degree of symptom of the patient, as well as the type and dose of another therapeutic agent used. Specifically, it can be administered, for example, by:
(1) intravenous administration: 1 to 5 mg/kg
(2) oral administration: 4 to 8 mg/kg

The "compound having an agonistic action on PPARγ" as used in the present description is not specifically limited, as long as it is a compound having an agonistic action on PPARγ, such as a compound represented by the formula (I) in WO 02/100812.

A preferred example of the compound having a PPARγ agonistic action means a compound selected from the group consisting of:
(1) 2-isopropoxy-3-[3-([4-(trifluoromethyl)benzyl]-oxyethanimidoyl)phenyl]propanoic acid,
(2) 3-{3-[3-(2,4-dichlorophenoxy)-2(S)-hydroxypropoxy]phenyl}-2(S)-isopropoxypropanoic acid,
(3) 3-(3-{2(R)-hydroxy-3-[4-chlorophenoxy]propoxy}phenyl)-2(S)-isopropoxypropanoic acid,
(4) 3-{3-[3-(2,4-dichlorophenoxy)-2(R)-hydroxypropoxy]phenyl}-2(S)-isopropoxypropanoic acid,
(5) 3-{3-[3-(2,4-dichlorophenoxy)-2(R)-fluoropropoxy]phenyl}-2(S)-isopropoxypropanoic acid,
(6) 3-{3-[3-(9-chlorophenoxy)-2(S)-fluoropropoxy]phenyl}-2(S)-isopropoxypropanoic acid,
(7) 3-{3-[3-(4-chloro-2-cyanophenoxy)-2(R)-fluoropropoxy]phenyl}-2(S)-isopropoxypropanoic acid,
(8) 3-{3-[3-(2,4-dichlorophenoxy)-2(S)-fluoropropoxy]-phenyl}-2(S)-isopropoxypropanoic acid,
(9) 3-{3-[3-(4-chlorophenoxy)-2(R)-fluoropropoxy]phenyl}-2(S)-isopropoxypropanoic acid,
(10) 3-{3-[3-(4-chloro-2-cyanophenoxy)-2(S)-fluoropropoxy]phenyl}-2(S)-isopropoxypropanoic acid,
(11) 2(S)-isopropoxy-3-{3-[(4-trifluoromethylbenzyloxycarbonylamino)methyl]phenyl}-propanoic acid,
(12) 2(S)-isopropoxy-3-{3-[(3-trifluoromethylbenzyloxycarbonylamino)methyl]phenyl}-propanoic acid,
(13) 2(S)-isopropoxy-3-{3-[(4-trifluoromethoxybenzyloxycarbonylamino)methyl]phenyl}-propanoic acid,
(14) 3-(3-{[3-trifluoromethoxybenzyloxycarbonylamino]-methyl}phenyl)-2(S)-isopropoxypropanoic acid,
(15) 3-{[3-(2,4-dichlorophenyl)carbamoyloxymethyl-4-ethoxy]phenyl}-2-isopropoxypropanoic acid,
(16) 3-({4-[5-(benzo[1,3]dioxolyl)]carbamoyloxymethyl}-phenyl)-2-isopropoxypropanoic acid,
(17) 3-{3-[3-(4-chlorophenyl)-2-propynyloxy]phenyl}-2(S)-isopropoxypropanoic acid,
(18) 3-{[3-(2,4-dichlorophenyl)carbamoyloxymethyl-4-ethoxy]phenyl}-2(S)-isopropoxypropanoic acid,
(19) 3-(3-{2(R)-hydroxy-3-[2-bromo-4-methylphenoxy]-propoxy}phenyl)-2(S)-isopropoxypropanoic acid,
(20) 3-{[4-(4-ethoxyphenyl)carbamoyloxymethyl]phenyl}-2-isopropoxypropanoic acid,
(21) 2-isopropoxy-3-[4-(2-{[4-(trifluoromethyl)phenyl]-carbamoyloxy}ethyl)phenyl]propanoic acid,
(22) 3-[3-([2,4-dichlorobenzoyl]aminomethyl)-4-methoxyphenyl]-2(S)-isopropoxypropanoic acid,
(23) 3-[3-([2-fluoro-4-(trifluoromethyl)benzoyl]-aminomethyl)-4-methoxyphenyl]-2(S)-isopropoxypropanoic acid,
(24) 2-ethoxy-3-(2-{2-[4-(trifluoromethyl)phenoxy]ethoxy}-4-pyridyl)propanoic acid,
(25) 3-(2-{2-[4-(tert-butyl)phenoxy]ethoxy}-4-pyridyl)-2-ethoxypropanoic acid,
(26) 3-(3-[(2-chloro-4-propoxybenzoyl)amino]methyl-2,4-dimethoxyphenyl)-2-isopropoxypropanoic acid,
(27) 3-(7-[(2,4-dichlorobenzoyl)amino]methylbenzo[b]furan-5-yl)-2-isopropoxypropanoic acid,
(28) 3-(7-[(2-chloro-4-propoxybenzoyl)amino]methyl-2,3-dihydrobenzo[b]furan-5-yl)-2-isopropoxypropanoic acid,
(29) 2-(3-{[(2-chloro-4-propoxybenzoyl)amino]methyl}-4-ethoxybenzyl)tetrahydro-2-furancarboxylic acid and
(30) 2-(3-{[(2-chloro-4-propoxybenzoyl)amino]methyl}-4-ethoxybenzyl)tetrahydro-2-furancarboxylic acid,
(31) pioglitazone,
(32) netoglitazone,
(33) tesaglitazar,
(34) 5-(2,4-dioxothiazolidin-5-ylmethyl)-2-methoxy-N-(4-trifluoromethylbenzyl)benzamide,
(35) 5-[4-(6-methoxy-1H-benzimidazol-2-ylmethoxy)benzyl]-thiazolidine-2,4-dione,
(36) 4-[4-(5-methyl-2-phenyloxazol-4-ylmethoxy)-benzyloxyimino]-4-phenylbutyric acid and
(37) (4-methoxyphenoxycarbonyl-[4-[2-(5-methyl-2-phenyloxazol-4-yl)ethoxy]benzyl]amino)butyric acid, a salt thereof or a hydrate of them.

As the preferred examples of the "compound having a PPARγ agonistic action", a compound selected from the group consisting of:
(1) 2-isopropoxy-3-[3-([4-(trifluoromethyl)benzyl]-oxyethanimidoyl)phenyl]propanoic acid,
(2) 3-{3-[3-(2,4-dichlorophenoxy)-2(R)-fluoropropoxy]-phenyl}-2(S)-isopropoxypropanoic acid,
(3) 3-{3-[3-(4-chlorophenoxy)-2(R)-fluoropropoxy]phenyl}-2(S)-isopropoxypropanoic acid,
(4) 3-{3-[3-(4-chloro-2-cyanophenoxy)-2(S)-fluoropropoxy]-phenyl}-2(S)-isopropoxypropanoic acid,
(5) 2(S)-isopropoxy-3-{3-[(4-trifluoromethoxybenzyloxycarbonylamino)methyl]phenyl}-propanoic acid,
(6) 3-(3-{[3-trifluoromethoxybenzyloxycarbonylamino]-methyl}phenyl)-2(S)-isopropoxypropanoic acid,
(7) pioglitazone,
(8) netoglitazone,
(9) tesaglitazar,
(10) 5-(2,4-dioxothiazolidin-5-ylmethyl)-2-methoxy-N-(4-trifluoromethylbenzyl)benzamide,
(11) 5-[4-(6-methoxy-1H-benzimidazol-2-ylmethoxy)benzyl]-thiazolidine-2,4-dione,
(12) 4-[4-(5-methyl-2-phenyloxazol-4-ylmethoxy)-benzyloxyimino]-4-phenylbutyric acid and
(13) (4-methoxyphenoxycarbonyl-[4-[2-(5-methyl-2-phenyloxazol-4-yl)ethoxy]benzyl]amino)acetic acid,
a salt thereof or a hydrate of them may be proposed.

As the preferred examples thereof, a compound selected from the group consisting of:
(1) 2-isopropoxy-3-[3-([4-(trifluoromethyl)benzyl]-oxyethanimidoyl)phenyl]propanoic acid,
(2) 3-{3-[3-(2,4-dichlorophenoxy)-2(S)-hydroxypropoxy]-phenyl}-2(S)-isopropoxypropanoic acid,
(3) 3-(3-{2(R)-hydroxy-3-[4-chlorophenoxy]propoxy}phenyl)-2(S)-isopropoxypropanoic acid,
(4) 3-{3-[3-(2,4-dichlorophenoxy)-2(R)-hydroxypropoxy]-phenyl}-2(S)-isopropoxypropanoic acid,
(5) 3-{3-[3-(2,4-dichlorophenoxy)-2(R)-fluoropropoxy]-phenyl}-2(S)-isopropoxypropanoic acid,
(6) 3-{3-[3-(4-chlorophenoxy)-2(S)-fluoropropoxy]phenyl}-2(S)-isopropoxypropanoic acid,
(7) 3-{3-[3-(4-chloro-2-cyanophenoxy)-2(R)-fluoropropoxy]phenyl}-2(S)-isopropoxypropanoic acid,
(8) 3-{3-[3-(2,4-dichlorophenoxy)-2(S)-fluoropropoxy]-phenyl}-2(S)-isopropoxypropanoic acid,
(9) 3-{3-[3-(4-chlorophenoxy)-2(R)-fluoropropoxy]phenyl}-2(S)-isopropoxypropanoic acid,
(10) 3-{3-[3-(4-chloro-2-cyanophenoxy)-2(S)-fluoropropoxy]phenyl}-2(S)-isopropoxypropanoic acid,
(11) 2(S)-isopropoxy-3-{3-[(4-trifluoromethylbenzyloxycarbonylamino)methyl]phenyl}-propanoic acid,
(12) 2(S)-isopropoxy-3-{3-[(3-trifluoromethylbenzyloxycarbonylamino)methyl]phenyl}-propanoic acid,
(13) 2(S)-isopropoxy-3-{3-[(4-trifluoromethoxybenzyloxycarbonylamino)methyl]phenyl}-propanoic acid,
(14) 3-(3-{[3-trifluoromethoxybenzyloxycarbonylamino]-methyl}phenyl)-2(S)-isopropoxypropanoic acid,
(15) 3-{[3-(2,4-dichlorophenyl)carbamoyloxymethyl-4-ethoxy]phenyl}-2-isopropoxypropanoic acid,
(16) 3-({9-[5-(benzo[1,3]dioxolyl)]carbamoyloxymethyl}-phenyl)-2-isopropoxypropanoic acid,
(17) 3-{3-[3-(4-chlorophenyl)-2-propynyloxy]phenyl}-2(S)-isopropoxypropanoic acid,
(18) 3-{[3-(2,4-dichlorophenyl)carbamoyloxymethyl-4-ethoxy]phenyl}-2(S)-isopropoxypropanoic acid,
(19) 3-(3-{2(R)-hydroxy-3-[2-bromo-4-methylphenoxy]-propoxy}phenyl)-2(S)-isopropoxypropanoic acid,
(20) 3-{[4-(4-ethoxyphenyl)carbamoyloxymethyl]phenyl}-2-isopropoxypropanoic acid,
(21) 2-isopropoxy-3-[4-(2-{[4-(trifluoromethyl)phenyl]-carbamoyloxy}ethyl)phenyl]propanoic acid,
(22) 3-[3-([2,4-dichlorobenzoyl]aminomethyl)-4-methoxyphenyl]-2(S)-isopropoxypropanoic acid,
(23) 3-[3-([2-fluoro-4-(trifluoromethyl)benzoyl]-aminomethyl)-4-methoxyphenyl]-2(S)-isopropoxypropanoic acid,
(24) 2-ethoxy-3-(2-{2-[4-(trifluoromethyl)phenoxy]ethoxy}-4-pyridyl)propanoic acid,
(25) 3-(2-{2-[4-(tert-butyl)phenoxy]ethoxy)-4-pyridyl)-2-ethoxypropanoic acid,
(26) 3-(3-[(2-chloro-4-propoxybenzoyl)amino]methyl-2,4-dimethoxyphenyl)-2-isopropoxypropanoic acid,
(27) 3-(7-[(2,4-dichlorobenzoyl)amino]methylbenzo[b]furan-5-yl)-2-isopropoxypropanoic acid,
(28) 3-(7-[(2-chloro-4-propoxybenzoyl)amino]methyl-2,3-dihydrobenzo[b]furan-5-yl)-2-isopropoxypropanoic acid,
(29) 2-(3-{[(2-chloro-4-propoxybenzoyl)amino]methyl}-4-ethoxybenzyl)tetrahydro-2-furancarboxylic acid and
(30) 2-(3-{[(2-chloro-4-propoxybenzoyl)amino]methyl}-4-ethoxybenzyl)tetrahydro-2-furancarboxylic acid, a salt thereof or a hydrate of them may be proposed.

As more preferred examples thereof, a compound selected from the group consisting of:
(1) 2-isopropoxy-3-[3-([4-(trifluoromethyl)benzyl]-oxyethanimidoyl)phenyl]propanoic acid,
(2) 3-{3-[3-(2,4-dichlorophenoxy)-2(R)-fluoropropoxy]phenyl}-2(S)-isopropoxypropanoic acid,
(3) 3-{3-[3-(4-chlorophenoxy)-2(R)-fluoropropoxy]phenyl}-2(S)-isopropoxypropanoic acid,
(4) 3-{3-[3-(4-chloro-2-cyanophenoxy)-2(S)-fluoropropoxy]phenyl}-2(S)-isopropoxypropanoic acid,
(5) 2(S)-isopropoxy-3-{3-[(4-trifluoromethoxybenzyloxycarbonylamino)methyl]phenyl}-propanoic acid and
(6) 3-(3-{[3-trifluoromethoxybenzyloxycarbonylamino]-methyl}phenyl)-2(S)-isopropoxypropanoic acid, a salt thereof or a hydrate of them may be proposed.

As further preferred examples thereof, a compound selected from the group consisting of:
3-{3-[3-(4-chloro-2-cyanophenoxy)-2(S)-fluoropropoxy]-phenyl}-2(S)-isopropoxypropanoic acid and
3-(3-{[3-trifluoromethoxybenzyloxycarbonylamino]methyl}-phenyl)-2(S)-isopropoxypropanoic acid, a salt thereof or a hydrate of them may be proposed.

As the most preferred example thereof, 3-{3-[3-(4-chloro-2-cyanophenoxy)-2(S)-fluoropropoxy]phenyl}-2(S)-isopropoxypropanoic acid, a salt thereof or a hydrate of them may be proposed.

Pioglitazone means a compound represented by the formula: 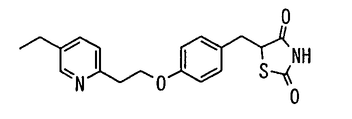 (Artneim. Forsch/Drug Res. 40 (I) p. 37 (1990)).

Netoglitazone means a compound represented by the formula: 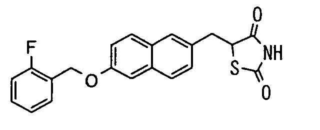 (EP-A1 604983 and JP-A 6-247945).

Tesaglitazar means a compound represented by the formula: 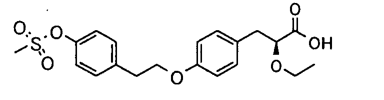 (WO 9962871).

5-(2,4-Dioxothiazolidin-5-ylmethyl)-2-methoxy-N-(4-trifluoromethylbenzyl)benzamide means a compound represented by the formula: 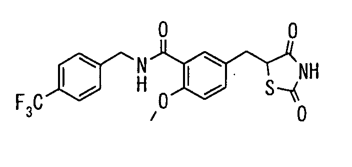 (J. Med. Chem. (37) 3977-3985 (1994)).

5-[4-(6-Methoxy-1H-benzimidazol-2-ylmethoxy)benzyl]-thiazolidine-2,4-dione means a compound represented by the formula: 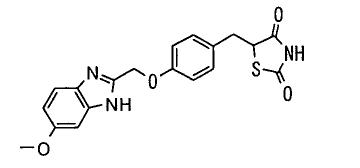 (EP-A1 0745600 and JP-A 2000-001487).

Synthetic methods of the following compounds (1) to (21) are described in WO 02/100812.
(1) 2-Isopropoxy-3-[3-([4-(trifluoromethyl)benzyl]-oxyethanimidoyl)phenyl]propanoic acid,
(2)3-{3-[3-(2,4-dichlorophenoxy)-2(S)-hydroxypropoxy]phenyl}-2(S)-isopropoxypropanoic acid,
(3) 3-(3-{2(R)-hydroxy-3-[4-chlorophenoxy]propoxy}phenyl)}-2(S)-isopropoxypropanoic acid,
(4) 3-{3-[3-(2,4-dichlorophenoxy)-2(R)-hydroxypropoxy]phenyl}-2(S)-isopropoxypropanoic acid,
(5) 3-{3-[3-(2,4-dichlorophenoxy)-2(R)-fluoropropoxy]phenyl}-2(S)-isopropoxypropanoic acid,
(6) 3-{3-[3-(4-chlorophenoxy)-2(S)-fluoropropoxy]phenyl}-2(S)-isopropoxypropanoic acid,
(7) 3-{3-[3-(4-chloro-2-cyanophenoxy)-2(R)-fluoropropoxy]phenyl}-2(S)-isopropoxypropanoic acid,
(8) 3-{3-[3-(2,4-dichlorophenoxy)-2(S)-fluoropropoxy]phenyl}-2(S)-isopropoxypropanoic acid,
(9) 3-{3-[3-(4-chlorophenoxy)-2(R)-fluoropropoxy]phenyl}-2(S)-isopropoxypropanoic acid,
(10) 3-{3-[3-(4-chloro-2-cyanophenoxy)-2(S)-fluoropropoxy]phenyl}-2(S)-isopropoxypropanoic acid,
(11) 2(S)-isopropoxy-3-{3-[(4-trifluoromethylbenzyloxycarbonylamino)methyl]phenyl}-propanoic acid,
(12) 2(S)-isopropoxy-3-{3-[(3-trifluoromethylbenzyloxycarbonylamino)methyl]phenyl}-propanoic acid,
(13) 2 (S)-isopropoxy-3-{3- [(4-trifluoromethoxybenzyloxycarbonylamino)methyl]phenyl}-propanoic acid,
(14) 3-(3-{[3-trifluoromethoxybenzyloxycarbonylamino]-methyl}phenyl)-2(S)-isopropoxypropanoic acid,
(15) 3-{[3-(2,4-dichlorophenyl)carbamoyloxymethyl-4-ethoxy] phenyl}-2-isopropoxypropanoic acid,
(16) 3-({4-[5-(benzo[1,3]dioxolyl)]carbamoyloxymethyl}-phenyl)-2-isopropoxypropanoic acid,
(17) 3-{3-[3-(4-chlorophenyl)-2-propynyloxy]phenyl}-2(S)-isopropoxypropanoic acid,
(18) 3-{[3-(2,4-dichlorophenyl)carbamoyloxymethyl-4-ethoxy]phenyl}-2(S)-isopropoxypropanoic acid,
(19) 3-(3-{2(R)-hydroxy-3-[2-bromo-4-methylphenoxy]propoxy}phenyl)-2(S)-isopropoxypropanoic acid,
(20) 3-{[4-(4-ethoxyphenyl)carbamoyloxymethyl]phenyl}-2-isopropoxypropanoic acid and
(21) 2-isopropoxy-3-[4-(2-{[4-(trifluoromethyl)phenyl]-carbamoyloxy}ethyl)phenyl]propanoic acid.

Synthetic methods of the following compounds (22) and (23) are described in PCT International Publication Number WO 01/25181.
(22) 3-[3-([2,4-Dichlorobenzoyl]aminomethyl)-4-methoxyphenyl]-2(S)-isopropoxypropanoic acid and
(23) 3-[3-([2-fluoro-4-(trifluoromethyl)benzoyl]-aminomethyl)-4-methoxyphenyl]-2(S)-isopropoxypropanoic acid.

Synthetic methods of the following compounds (24) and (25) are described in WO 02/79162.
(24) 2-Ethoxy-3-(2-{2-[4-(trifluoromethyl)phenoxy]ethoxy}-4-pyridyl)propanoic acid and
(25) 3-(2-{2-[4-(tert-butyl)phenoxy]ethoxy}-4-pyridyl)-2-ethoxypropanoic acid.

Synthetic methods of the following compounds (26) to (28) are described in WO 02/81428.
(26) 3-(3-[(2-Chloro-4-propoxybenzoyl)amino]methyl-2,4-dimethoxyphenyl)-2-isopropoxypropanoic acid,
(27) 3-(7-[(2,4-Dichlorobenzoyl)amino]methylbenzo[b]furan-5-yl)-2-isopropoxypropanoic acid and
(28) 3-(7-[(2-chloro-4-propoxybenzoyl)amino]methyl-2,3-dihydrobenzo[b]furan-5-yl)-2-isopropoxypropanoic acid.

Synthetic methods of the following compounds (29) and (30) are described in WO 03/16265.
(29) 2-(3-{[(2-Chloro-4-propoxybenzoyl)amino]methyl}-4-ethoxybenzyl)tetrahydro-2-furancarboxylic acid and
(30) 2-(3-{[(2-chloro-4-propoxybenzoyl)amino]methyl}-4-ethoxybenzyl)tetrahydro-2-furancarboxylic acid.

As the "salt" as described in the present specification, for example, salts with inorganic acids; salts with organic acids; salts with inorganic bases; salts with organic bases; and salts with acidic or basic amino acids, of which pharmacologically acceptable salts are preferred. The acid or base may form a salt in an appropriate ratio of 0.1 to 5 molecules to one molecule of the compound in question.

Preferred examples of the salts with inorganic acids are salts with hydrochloric acid, hydrobromic acid, sulfuric acid, nitric acid or phosphoric acid. Preferred examples of the salts with organic acids are salts with acetic acid, succinic acid, fumaric acid, maleic acid, tartaric acid, citric acid, lactic acid, stearic acid, benzoic acid, methanesulfonic acid or p-toluenesulfonic acid.

Preferred examples of the salts with inorganic bases are alkali metal salts such as sodium salts and potassium salts; alkaline earth metal salts such as calcium salts and magnesium salts; aluminum salts; and ammonium salts. Preferred examples of the salts with organic bases are diethylamine salts, diethanolamine salts, meglumine salts and N,N'-dibenzylethylenediamine salts.

Preferred examples of the salts with acidic amino acids are aspartates and glutamates. Preferred examples of the salts with basic amino acids are arginine salts, lysine salts and ornithine salts.

The agent for treating an inflammatory bowel disease of the present invention containing a compound having a PPARγ agonistic action and another compound having an anti-inflammatory action (e.g., an aminosalicylic acid derivative, an anti-inflammatory glucocorticoid, a compound having an immunosuppressive action, an anti-TNFsα antibody, a pituitary hormone or a compound having an anti-infective action) (1) exhibits excellent treatment effects superior to those of the single administration of a compound having a PPARγ agonistic action or of the single administration of another agent for treating an inflammatory bowel disease and (2) has a possibility of mitigating adverse drug actions derived from the PPARγ agonist or the other agent for treating an inflammatory bowel disease. The agent has been found to be a medicament having satisfactory effects on inflammatory bowel diseases such as ulcerative colitis or Crohn's disease.

### Examples

### Experimental Method

A total of 0.08 ml of a 1:1 (v/v) mixture of 10% picrylsulfonic acid (TNBS) solution and ethanol was administered to anesthetized male Balb/c mice (Charles River Japan, Inc., Yokohama, Japan) according to previous reports (J Exp Med 2001; 193: p827-38, J Exp Med 2001; 193: p25-34) to thereby induce experimental colitis. The colon (large intestine) was sampled two days later, and the length of a flare site accompanied with ulcer or hyperplasia near to the lumen was measured and was defined as the lesion length. The inhibition (%) was calculated from the average lesion length of a treated group, compared with the average lesion length of a control group. The test compound suspended in a 0.5% solution of methylcellulose was orally administered to the mice via sonde once a day from two days before the beginning of colitis induction.

### Compound 1 means 3-(3-{[3-trifluoromethoxybenzyloxycarbonylamino]methyl}phenyl)-2(S)-isopropoxypropanoic acid.

### Results

Table 1 shows the results of single administration of rosiglitazone.
Table 2 shows the results of administration of Compound 1.
Table 3 shows the results of single administration of sulfasalazine.
Table 4 shows the results of single administration of cyclosporin.
Table 5 shows the results of combination administration of Compound 1, sulfasalazine and/or cyclosporin.

**Table 1**

| Rosiglitazone (mg/kg/day) | Inhibition (%) |
|---|---|
| 1 | 23 |
| 10 | 37 |

**Table 2**

| Compound 1 (mg/kg/day) | Inhibition (%) |
|---|---|
| 0.3 | 8 |
| 1 | 21 |
| 3 | 38 |
| 10 | 34 |

**Table 3**

| Sulfasalazine (mg/kg/day) | Inhibiton (%) |
|---|---|
| 100 | 40 |
| 300 | 35 |

**Table 4**

| Cyclosporin (mg/kg/day) | Inhibiton (%) |
|---|---|
| 20 | 54 |
| 30 | 57 |

**Table 5**

| Compound 1 (3 mg/kg/day) | Sulfasalazine (100 mg/kg/day) | Cyclosporin (20 mg/kg/day) | Inhibiton (%) |
|---|---|---|---|
| (+) | (-) | (-) | 29 |
| (-) | (+) | (-) | 43 |
| (-) | (-) | (+) | 54 |
| (+) | (+) | (-) | 71 |
| (+) | (-) | (+) | 71 |

These results demonstrate the following points.
(1) Single administration of rosiglitazone or Compound 1 as a PPARγ agonist, or sulfasalazine or cyclosporin partially improves colitis.
(2) Combination administration of Compound 1 and sulfasalazine, or combination administration of Compound 1 and cyclosporin improves colitis more than the single administration of each of the three test compounds.
(3) Single administration of Compound 1, sulfasalazine or cyclosporin at a dose higher than that in the combination administration improves colitis less effectively than that of the combination administration of Compound 1 and sulfasalazine or of Compound 1 and cyclosporin.

## Claims

1. An agent for treating an inflammatory bowel disease comprising a combination of (a) at least one compound having an anti-inflammatory action and being selected from the group consisting of an aminosalicylic acid derivative, an anti-inflammatory glucocorticoid, a compound having an immunosuppressive action, an anti-TNFα antibody, a pituitary hormone and a compound having an anti-infective action as an active ingredient and (b) at least one compound having a PPARγ agonistic action as an active ingredient, wherein the agent is so configured that the compound (a) and the compound (b) are used simultaneously, separately or every scheduled time.

2. An agent for treating an inflammatory bowel disease comprising a combination of (a) at least one compound having an anti-inflammatory action and being selected from the group consisting of an aminosalicylic acid derivative, an anti-inflammatory glucocorticoid, a compound having an immunosuppressive action and an anti-TNFα antibody as an active ingredient and (b) at least one compound having a PPARγ agonistic action as an active ingredient, wherein the agent is so configured that the compound (a) and the compound (b) are used simultaneously, separately or every scheduled time.

3. An agent for treating an inflammatory bowel disease comprising a combination of (a) at least one compound having an anti-inflammatory action and being selected from the group consisting of an aminosalicylic acid derivative and a compound having an immunosuppressive action as an active ingredient and (b) at least one compound having a PPARγ agonistic action as an active ingredient, wherein the agent is so configured that the compound (a) and the compound (b) are used simultaneously, separately or every scheduled time.

4. The agent for treating an inflammatory bowel disease according to claim 1, wherein the compound having a PPARγ agonistic action is a compound having a PPARγ agonistic action for use in leukocytapheresis treatment or granulocytapheresis treatment.

5. The agent for treating an inflammatory bowel disease according to any one of claims 1 to 4, wherein the compound having a PPARγ agonistic action is a compound selected from the group consisting of:
(1) 2-isopropoxy-3-[3-([4-(trifluoromethyl)benzyl]-oxyethanimidoyl)phenyl]propanoic acid,
(2) 3-{3-[3-(2,4-dichlorophenoxy)-2(S)-hydroxypropoxy]phenyl}-2(S)-isopropoxypropanoic acid,
(3) 3-(3-{2(R)-hydroxy-3-[4-chlorophenoxy]propoxy}phenyl)-2(S)-isopropoxypropanoic acid,
(4) 3-{3-[3-(2,4-dichlorophenoxy)-2(R)-hydroxypropoxy]phenyl}-2(S)-isopropoxypropanoic acid,
(5) 3-{3-[3-(2,4-dichlorophenoxy)-2(R)-fluoropropoxy]phenyl}-2(S)-isopropoxypropanoic acid,
(6) 3-{3-[3-(4-chlorophenoxy)-2(S)-fluoropropoxy]phenyl}-2(S)-isopropoxypropanoic acid,
(7) 3-{3-[3-(4-chloro-2-cyanophenoxy)-2(R)-fluoropropoxy]phenyl}-2(S)-isopropoxypropanoic acid,
(8) 3-{3-[3-(2,4-dichlorophenoxy)-2(S)-fluoropropoxy]phenyl}-2(S)-isopropoxypropanoic acid,
(9) 3-{3-[3-(9-chlorophenoxy)-2(R)-fluoropropoxy]phenyl}-2(S)-isopropoxypropanoic acid,
(10) 3-{3-[3-(4-chloro-2-cyanophenoxy)-2(S)-fluoropropoxy]phenyl}-2(S)-isopropoxypropanoic acid,
(11) 2(S)-isopropoxy-3-{3-[(4-trifluoromethylbenzyloxycarbonylamino)methyl]phenyl}-propanoic acid,
(12) 2(S)-isopropoxy-3-{3-[(3-trifluoromethylbenzyloxycarbonylamino)methyl]phenyl}-propanoic acid,
(13) 2(S)-isopropoxy-3-{3-[(4-trifluoromethoxybenzyloxycarbonylamino)methyl]phenyl}-propanoic acid,
(14) 3-(3-{[3-trifluoromethoxybenzyloxycarbonylamino]-methyl}phenyl)-2(S)-isopropoxypropanoic acid,
(15) 3-{[3-(2,4-dichlorophenyl)carbamoyloxymethyl-4-ethoxy]phenyl}-2-isopropoxypropanoic acid,
(16) 3-({4-[5-(benzo[1,3]dioxolyl)]carbamoyloxymethyl}-phenyl)-2-isopropoxypropanoic acid,
(17) 3-{3-[3-(9-chlorophenyl)-2-propynyloxy]phenyl}-2(S)-isopropoxypropanoic acid,
(18) 3-{[3-(2,4-dichlorophenyl)carbamoyloxymethyl-4-ethoxy]phenyl}-2(S)-isopropoxypropanoic acid,
(19) 3-(3-{2(R)-hydroxy-3-[2-bromo-4-methylphenoxy]-propoxy}phenyl)-2(S)-isopropoxypropanoic acid,
(20) 3-{[9-(9-ethoxyphenyl)carbamoyloxymethyl]phenyl}-2-isopropoxypropanoic acid,
(21) 2-isopropoxy-3-[4-(2-{[4-(trifluoromethyl)phenyl]-carbamoyloxy}ethyl)phenyl]propanoic acid,
(22) 3-[3-([2,4-dichlorobenzoyl]aminomethyl)-4-methoxyphenyl]-2(S)-isopropoxypropanoic acid,
(23) 3-[3-([2-fluoro-4-(trifluoromethyl)benzoyl]-aminomethyl)-4-methoxyphenyl]-2(S)-isopropoxypropanoic acid,
(24) 2-ethoxy-3-(2-{2-[4-(trifluoromethyl)phenoxy]ethoxy}-4-pyridyl)propanoic acid,
(25) 3-(2-{2-[4-(tert-butyl)phenoxy]ethoxy}-4-pyridyl)-2-ethoxypropanoic acid,
(26) 3-(3-[(2-chloro-4-propoxybenzoyl)amino]methyl-2,4-dimethoxyphenyl)-2-isopropoxypropanoic acid,
(27) 3-(7-[(2,4-dichlorobenzoyl)amino]methylbenzo[b]furan-5-yl)-2-isopropoxypropanoic acid,
(28) 3-(7-[(2-chloro-4-propoxybenzoyl)amino]methyl-2,3-dihydrobenzo[b]furan-5-yl)-2-isopropoxypropanoic acid,
(29) 2-(3-{[(2-chloro-4-propoxybenzoyl)amino]methyl}-4-ethoxybenzyl)tetrahydro-2-furancarboxylic acid,
(30) 2-(3-{[(2-chloro-4-propoxybenzoyl)amino]methyl}-4-ethoxybenzyl)tetrahydro-2-furancarboxylic acid,
(31) pioglitazone,
(32) netoglitazone,
(33) tesaglitazar,
(34) 5-(2,4-dioxothiazolidin-5-ylmethyl)-2-methoxy-N-(4-trifluoromethylbenzyl)benzamide,
(35) 5-[4-(6-methoxy-1H-benzimidazol-2-ylmethoxy)benzyl]-thiazolidine-2,4-dione,
(36) 4-[4-(5-methyl-2-phenyloxazol-4-ylmethoxy)-benzyloxyimino]-4-phenylbutyric acid and
(37) (4-methoxyphenoxycarbonyl-[4-[2-(5-methyl-2-phenyloxazol-4-yl)ethoxy]benzyl]amino)acetic acid, a salt thereof or a hydrate of them.

6. The agent for treating an inflammatory bowel disease according to any one of claims 1 to 4, wherein the compound having a PPARγ agonistic action is a compound selected from the group consisting of:
(1) 2-isopropoxy-3-[3-([4-(trifluoromethyl)benzyl]-oxyethanimidoyl)phenyl]propanoic acid,
(2) 3-{3-[3-(2,4-dichlorophenoxy)-2(R)-fluoropropoxy]phenyl}-2(S)-isopropoxypropanoic acid,
(3) 3-{3-[3-(4-chlorophenoxy)-2(R)-fluoropropoxy]phenyl}-2(S)-isopropoxypropanoic acid,
(4) 3-{3-[3-(4-chloro-2-cyanophenoxy)-2(S)-fluoropropoxy] phenyl}-2(S)-isopropoxypropanoic acid,
(5) 2(S)-isopropoxy-3-{3-[(4-trifluoromethoxybenzyloxycarbonylamino)methyl]phenyl}-propanoic acid,
(6) 3-(3-{[3-trifluoromethoxybenzyloxycarbonylamino]-methyl}phenyl)-2(S)-isopropoxypropanoic acid,
(7) pioglitazone,
(8) netoglitazone,
(9) tesaglitazar,
(10) 5-(2,4-dioxothiazolidin-5-ylmethyl)-2-methoxy-N-(4-trifluoromethylbenzyl)benzamide,
(11) 5-[4-(6-methoxy-1H-benzimidazol-2-ylmethoxy)benzyl]-thiazolidine-2,4-dione,
(12) 4-[4-(5-methyl-2-phenyloxazol-4-ylmethoxy)-benzyloxyimino]-4-phenylbutyric acid and
(13) (4-methoxyphenoxycarbonyl-[4-[2-(5-methyl-2-phenyloxazol-4-yl)ethoxy]benzyl]amino)acetic acid, a salt thereof or a hydrate of them.

7. The agent for treating an inflammatory bowel disease according to any one of claims 1 to 4, wherein the compound having a PPARγ agonistic action is a compound selected from the group consisting of:
(1) 2-isopropoxy-3-[3-([4-(trifluoromethyl)benzyl]-oxyethanimidoyl)phenyl]propanoic acid,
(2) 3-{3-[3-(2,4-dichlorophenoxy)-2(S)-hydroxypropoxy]phenyl}-2(S)-isopropoxypropanoic acid,
(3) 3-(3-{2(R)-hydroxy-3-[4-chlorophenoxy]propoxy}phenyl)-2(S)-isopropoxypropanoic acid,
(4) 3-{3-[3-(2,4-dichlorophenoxy)-2(R)-hydroxypropoxy]phenyl}-2(S)-isopropoxypropanoic acid,
(5) 3-{3-[3-(2,4-dichlorophenoxy)-2(R)-fluoropropoxy]phenyl}-2(S)-isopropoxypropanoic acid,
(6) 3-{3-[3-(4-chlorophenoxy)-2(S)-fluoropropoxy]phenyl}-2(S)-isopropoxypropanoic acid,
(7) 3-{3-[3-(4-chloro-2-cyanophenoxy)-2(R)-fluoropropoxy]phenyl}-2(S)-isopropoxypropanoic acid,
(8) 3-{3-[3-(2,4-dichlorophenoxy)-2(S)-fluoropropoxy] phenyl}-2(S)-isopropoxypropanoic acid,
(9) 3-{3-[3-(4-chlorophenoxy)-2(R)-fluoropropoxy]phenyl}-2(S)-isopropoxypropanoic acid,
(10) 3-{3-[3-(4-chloro-2-cyanophenoxy)-2(S)-fluoropropoxy]-phenyl}-2(S)-isopropoxypropanoic acid,
(11) 2(S)-isopropoxy-3-{3-[(4-trifluoromethylbenzyloxycarbonylamino)methyl]phenyl}-propanoic acid,
(12) 2(S)-isopropoxy-3-{3-[(3-trifluoromethylbenzyloxycarbonylamino)methyl]phenyl}-propanoic acid,
(13) 2(S)-isopropoxy-3-{3-[(4-trifluoromethoxybenzyloxycarbonylamino)methyl]phenyl}-propanoic acid,
(14) 3-(3-{[3-trifluoromethoxybenzyloxycarbonylamino]-methyl}phenyl)-2(S)-isopropoxypropanoic acid,
(15) 3-{[3-(2,4-dichlorophenyl)carbamoyloxymethyl-4-ethoxy]phenyl}-2-isopropoxypropanoic acid,
(16) 3-({4-[5-(benzo[1,3]dioxolyl)]carbamoyloxymethyl}-phenyl)-2-isopropoxypropanoic acid,
(17) 3-{3-[3-(4-chlorophenyl)-2-propynyloxy]phenyl}-2(S)-isopropoxypropanoic acid,
(18) 3-{[3-(2,4-dichlorophenyl)carbamoyloxymethyl-4-ethoxy]phenyl}-2(S)-isopropoxypropanoic acid,
(19) 3-(3-{2(R)-hydroxy-3-[2-bromo-4-methylphenoxy]propoxy}phenyl)-2(S)-isopropoxypropanoic acid,
(20) 3-{[4-(4-ethoxyphenyl)carbamoyloxymethyl]phenyl}-2-isopropoxypropanoic acid,
(21) 2-isopropoxy-3-[4-(2-{[4-(trifluoromethyl)phenyl]-carbamoyloxy}ethyl)phenyl]propanoic acid,
(22) 3-[3-([2,4-dichlorobenzoyl]aminomethyl)-4-methoxyphenyl]-2(S)-isopropoxypropanoic acid,
(23) 3-[3-([2-fluoro-4-(trifluoromethyl)benzoyl]-aminomethyl)-4-methoxyphenyl]-2(S)-isopropoxypropanoic acid,
(24) 2-ethoxy-3-(2-{2-[4-(trifluoromethyl)phenoxy]ethoxy}-4-pyridyl)propanoic acid,
(25) 3-(2-{2-[4-(tert-butyl)phenoxy]ethoxy}-4-pyridyl)-2-ethoxypropanoic acid,
(26) 3-(3-[(2-chloro-4-propoxybenzoyl)amino]methyl-2,4-dimethoxyphenyl)-2-isopropoxypropanoic acid,
(27) 3-(7-[(2,4-dichlorobenzoyl)amino]methylbenzo[b]furan-5-yl)-2-isopropoxypropanoic acid,
(28) 3-(7-[(2-chloro-4-propoxybenzoyl)amino]methyl-2,3-dihydrobenzo[b]furan-5-yl)-2-isopropoxypropanoic acid,
(29) 2-(3-{[(2-chloro-4-propoxybenzoyl)amino]methyl}-4-ethoxybenzyl)tetrahydro-2-furancarboxylic acid and
(30) 2-(3-{[(2-chloro-4-propoxybenzoyl)amino]methyl}-4-ethoxybenzyl)tetrahydro-2-furancarboxylic acid, a salt thereof or a hydrate of them.

8. The agent for treating an inflammatory bowel disease according to any one of claims 1 to 4, wherein the compound having a PPARγ gonistic action is a compound selected from the group consisting of:
(1) 2-isopropoxy-3-[3-([4-(trifluoromethyl)benzyl]-oxyethanimidoyl)phenyl]propanoic acid,
(2) 3-{3-[3-(2,4-dichlorophenoxy)-2(R)-fluoropropoxy]phenyl}-2(S)-isopropoxypropanoic acid,
(3) 3-{3-[3-(4-chlorophenoxy)-2(R)-fluoropropoxy]phenyl}-2(S)-isopropoxypropanoic acid,
(4) 3-{3-[3-(4-chloro-2-cyanophenoxy)-2(S)-fluoropropoxy]phenyl}-2(S)-isopropoxypropanoic acid,
(5) 2(S)-isopropoxy-3-{3-[(4-trifluoromethoxybenzyloxycarbonylamino)methyl]phenyl}-propanoic acid and
(6) 3-(3-{[3-trifluoromethoxybenzyloxycarbonylamino]-methyl}phenyl)-2(S)-isopropoxypropanoic acid, a salt thereof or a hydrate of them.

9. The agent for treating an inflammatory bowel disease according to any one of claims 1 to 4, wherein the compound having a PPARγ agonistic action is 3-{3-[3-(4-chloro-2-cyanophenoxy)-2(S)-fluoropropoxy]phenyl}-2(S)-isopropoxypropanoic acid, a salt thereof or a hydrate of them.

10. The agent for treating an inflammatory bowel disease according to any one of claims 1 to 3, wherein the aminosalicylic acid derivative is:
(1) sulfasalazine,
(2) mesalazine,
(3) olsalazine or
(4) balsalazide.

11. The agent for treating an inflammatory bowel disease according to claim 1 or 2, wherein the anti-inflammatory glucocorticoid is:
(1) prednisolone,
(2) betamethasone,
(3) hydrocortisone,
(4) cortisone acetate,
(5) methylprednisolone,
(6) prednisone or
(7) budesonide.

12. The agent for treating an inflammatory bowel disease according to any one of claims 1 to 3, wherein the compound having an immunosuppressive action is:
(1) cyclosporin,
(2) azathioprine,
(3) 6-mercaptopurine,
(4) tacrolimus or
(5) methotrexate.

13. The agent for treating an inflammatory bowel disease according to claim 1 or 2, wherein the anti-TNFα antibody is an antibody contained in:
(1) infliximab,
(2) etanercept,
(3) CDP-571,
(4) adalimumab or
(5) CDP-870.

14. The agent for treating an inflammatory bowel disease according to claim 1, wherein the compound having an anti-infective action is:
(1) metronidazole,
(2) clarithromycin,
(3) tobramycin,
(4) ciprofloxacin hydrochloride,
(5) ampicillin,
(6) cefazolin,
(7) ofloxacine or
(8) levofloxacin.

15. The agent for treating an inflammatory bowel disease according to claim 1, wherein the pituitary hormone is tetracosactide acetate.

16. The agent for treating an inflammatory bowel disease according to claim 2, wherein the group consisting of an aminosalicylic acid derivative, an anti-inflammatory glucocorticoid, a compound having an immunosuppressive action and an anti-TNFα antibody is the group consisting of sulfasalazine, mesalazine, prednisolone, betamethasone, hydrocortisone, cortisone acetate, methylprednisolone, prednisone, cyclosporin and tacrolimus.

17. The agent for treating an inflammatory bowel disease according to claim 3, wherein one compound having an anti-inflammatory action and being selected from the group consisting of an aminosalicylic acid derivative and a compound having an immunosuppressive action is sulfasalazine or cyclosporin.

18. An agent for treating an inflammatory bowel disease, which comprises a combination of (a) a compound having an anti-inflammatory action and being selected from the group consisting of sulfasalazine and cyclosporin as an active ingredient and (b) a compound having a PPARγ agonistic action and being 3-{3-[3-(4-chloro-2-cyanophenoxy)-2(S)-fluoropropoxy]phenyl}-2(S)-isopropoxypropanoic acid, a salt thereof or a hydrate of them as an active ingredient, wherein the agent is so configured that the compound (a) and the compound (b) are used simultaneously, separately or every scheduled time.

19. The agent according to any one of claims 1 to 17, wherein the inflammatory bowel disease is ulcerative colitis.

20. The agent according to any one of claims 1 to 17, wherein the inflammatory bowel disease is Crohn's disease.

21. An agent for treating an inflammatory bowel disease, which comprises a combination of the compound (a) and the compound (b) as descried in claim 1.

22. The agent according to claim 21, wherein the combination of the compound (a) and the compound (b) is simultaneous or separate administration.

23. The agent according to claim 21, wherein the combination of the compound (a) and the compound (b) is successive administration.

24. Use of the combination of the compound (a) and the compound (b) as described in claim 1, for producing an agent for treating an inflammatory bowel disease.

25. The use according to claim 24, wherein the combination of the compound (a) and the compound (b) is simultaneous or separate administration.

26. The use according to claim 24, wherein the combination of the compound (a) and the compound (b) is administration every scheduled time.

27. A method for treating an inflammatory bowel disease, which comprises administering a pharmacologically effective amount of the combination of the compound (a) and the compound (b) described in claim 1 to a patient.

28. The method according to claim 27, which comprises administering the compound (a) and the compound (b) simultaneously or separately to the patient.

29. The method according to claim 27, which comprises administering the compound (a) and the compound (b) every scheduled time to the patient.
